# EUROPEAN PATENT APPLICATION

(11) **EP 4 571 773 A1**
(43) Date of publication of application: **18.06.2025**
(21) Application number: 23216596.9
(22) Date of filing: 14.12.2023
(51) Int. Cl.: G16H 40/63, A61B 5/11, G16H 80/00

(54) **CONSTRUCTION OF MEDICAL MESSAGES**

(71) Applicant: Koninklijke Philips N.V., 5656 AG Eindhoven (NL)
(72) Inventor: MUIJLWIJK, Heleen, Eindhoven (NL); VAN EE, Raymond, Eindhoven (NL); WESTERINK, Joanne Henriëtte Desirée Monique, 5656AG Eindhoven (NL); MENA BENITO, Maria Estrella, Eindhoven (NL); JELFS, Sam Martin, Eindhoven (NL)
(74) Representative: Philips Intellectual Property & Standards

(57) **Abstract**

Disclosed herein is a medical system (100, 300, 500). The medical system comprises a memory (110) storing machine executable instructions (120). The medical system further comprises a computational system (104). Execution of the machine executable instructions causes the computational system to: receive (204) subject sensor data (128) descriptive of a subject; receive (206) a message text (130) at least partially via a user interface; record (208) message metadata (134) descriptive of entry of the message text using the user interface; generate (210) a control signal (138) by comparing the subject sensor data and the message metadata to a predetermined criterion, wherein the control signal is configured to modify the user interface for further entry or modification of the message text; and construct (212) a medical message (140) using the message text.

## Description

### TECHNICAL FIELD

The invention relates to medical communication, in particular to the construction of medical messages.

### BACKGROUND

Communication can best be summarized as the transmission of a message from a sender to a receiver in an understandable manner. Effective communication means sharing of a common meaning between the sender and receiver, that is, effective communication leads to understanding the meaning of the message and how it is intended by the sender.

The journal article Hohenstein, et. al,. et al. "Artificial intelligence in communication impacts language and social relationships," Sci Rep 13, 5487 (2023), https://doi.org/10.1038/s41598-023-30938-9 discloses that Artificial Intelligence (AI) is already widely used in daily communication, but despite concerns about Al's negative effects on society the social consequences of using it to communicate remain largely unexplored. This article investigates the social consequences of one of the most pervasive Al applications, algorithmic response suggestions ("smart replies"), which are used to send billions of messages each day. Two randomized experiments provide evidence that these types of algorithmic recommender systems change how people interact with and perceive one another in both pro-social and anti-social ways. This article discloses that using algorithmic responses changes language and social relationships. More specifically, it increases communication speed, use of positive emotional language, and conversation partners evaluate each other as closer and more cooperative. However, consistent with common assumptions about the adverse effects of AI, people are evaluated more negatively if they are suspected to be using algorithmic responses. Thus, even though AI can increase the speed of communication and improve interpersonal perceptions, the prevailing anti-social connotations of AI undermine these potential benefits if used overtly.

### SUMMARY

The invention provides for a medical system, a computer program, and a method in the independent claims. Embodiments are given in the dependent claims.

In one aspect the invention provides for a medical system that comprises a memory storing machine-executable instructions. The medical system further comprises a computational system. Execution of the machine-executable instructions causes the computational system to receive subject sensor data descriptive of a subject. Execution of the machine-executable instructions further causes the computational system to receive a message text at least partially via a user interface. Execution of the machine-executable instructions further causes the computational system to record message metadata descriptive of entry of the message text using the user interface. Execution of the machine-executable instructions further causes the computational system to generate a control signal by comparing the subject sensor data and the message metadata to a predetermined criterion. The control signal is configured to modify the user interface for further entry or modification of the message text. Execution of the machine-executable instructions further causes the computational system to construct a medical message using the message text.

In another aspect the invention further provides for a method. The method comprises receiving subject sensor data descriptive of a subject. The method further comprises receiving a message text at least partially via a user interface. The method further comprises recording message metadata that is descriptive of entry of the message text using the user interface. The method further comprises generating a control signal by comparing the subject sensor data and the message metadata to a predetermined criterion. The control signal is configured to modify the user interface for further entry or modification of the message text. The method further comprises constructing a medical message using the message text.

In another aspect the invention further provides for a computer program that comprises machine-executable instructions. Execution of the machine-executable instructions causes the computational system to receive subject sensor data that is descriptive of a subject. Execution of the machine-executable instructions further causes the computational system to receive a message text at least partially via the user interface. Execution of the machine-executable instructions further causes the computational system to record message metadata descriptive of entry of the message text using the user interface. Execution of the machine-executable instructions further causes the computational system to generate a control signal by comparing the subject sensor data and the message metadata to a predetermined criterion. The control signal is configured to modify the user interface for further entry or modification of the message text. Execution of the machine-executable instructions further causes the computational system to construct a medical message using the message text.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 3.
Fig. 5 illustrates a further example of a medical system.

### DESCRIPTION OF EMBODIMENTS

Like numbered elements in these figures are either equivalent elements or perform the same function. Elements which have been discussed previously will not necessarily be discussed in later figures if the function is equivalent.

When a message is communicated from a sender to a receiver, and this communication is delayed or remote, often it is unclear for the receiver under which circumstances and context the message was composed by the sender. This can hinder accurate interpretation of the message because it is not only the message itself, but also the context under which it is generated that is essential for a correct understanding of what is intended. Examples may provide for an improved means of generating a medical message by using a control signal generated using subject sensor data (sensor data measured from the intended recipient of the message) and message metadata that is descriptive of the entry of a message text used in constructing the medical message.

Examples provide for a medical system that comprises a memory storing machine-executable instructions. The medical system further comprises a computational system. The computational system may refer to one or more computational systems or computing cores that are located at one or more locations. Execution of the machine-executable instructions causes the computational system to receive subject sensor data descriptive of a subject. The subject sensor data may for example comprise sensor data that was measured and is descriptive of a physical property or action of the subject. In some examples the subject sensor data may be received in real time or as a subject sensor is measuring the subject sensor data. Execution of the machine-executable instructions further causes the computational system to receive a message text at least partially via a user interface. A user interface, as used herein, encompasses a control surface or human interface device which enables an operator to control and operate a computer and perform such tasks as entering message text. In some examples the message text is typed or entered completely by the user. In other examples the message text may be partially generated or provided via a template.

Execution of the machine-executable instructions further causes the computational system to record message metadata that is descriptive of entry of the message text using the user interface. This may for example include such things as metadata descriptive of how the user interface was used. This may include such things as how quickly the message text was entered as well as how much time was spent revising or changing the message text. Execution of the machine-executable instructions further causes the computational system to generate a control signal by comparing the subject sensor data and the message metadata to a predetermined criterion. For example, the subject sensor data and the message metadata may be used to look up a control signal in a lookup table. In other examples there may be more complicated database systems, expert systems or other information retrieval systems which are used to provide the predetermined criterion. The control signal is configured to modify the user interface for further entry or modification of the message text. The control signal may for example be used to modify the user interface as the operator is using it. This may for example force the operator to revise or revisit a portion of the message text. In other examples it may for example detect an unfavorable condition where the operator is angry or provides an indication or did not spend enough time editing the text. In this case for example the control signal may be used to modify the user interface so that the operator is not able to send the message or must revise certain portions of the message text or text further. Execution of the machine-executable instructions further causes the computational system to construct a medical message using the message text.

Examples may have the benefit that both the subject sensor and the message metadata is used to control the user interface and/or to modify the message text. This may have the benefit of adjusting the medical message such that it better fits the subject as is described by the state of the subject in the subject sensor data.

In another example, execution of the machine-executable instructions further causes the computational system to transmit the medical message to the subject. This may for example be transmitting the medical message via a network connection to a different computer or even, for example, a mobile device of the subject. This example may be beneficial because the use of the subject sensor data and the message metadata may provide for better control of sending of the medical message to the subject.

In another example, the medical system further comprises a subject display unit. The subject display unit may for example be a computer display or a display on a handheld computing device among other things. The transmitting of the medical message to the subject comprises displaying the medical message on the subject display unit. Execution of the machine-executable instructions further causes the computational system to generate display configuration commands for the subject display unit using the control signal. Execution of the machine-executable instructions further causes the computational system to append the display configuration commands to the medical message. The subject display is configured to modify the display of the medical message using the display configuration commands. This example may be beneficial because then the display of the medical message is automatically configured according to the subject sensor data that was measured from the subject. This for example may be beneficial in presenting the medical message to the subject in a way which may better fit the subconscious state of the subject.

In another example the medical system further comprises a subject sensor system configured for measuring the subject sensor data and for transmitting the subject sensor data to the computational system. This example may be beneficial because it may provide for a means of automatically collecting the subject sensor data and providing it to the computational system for immediate use.

In another example the subject sensor data comprises subject eye motion data that is descriptive of reading by the subject. The subject sensor system may then for example provide for a system for tracking the eye motion of the subject.

The eye tracking system may for example be a commercially available system which measures where a user is looking or their point of gaze. Such systems are known and may operate using different principles such as an attachment to the eye such as an embedded mirror or magnetic sensor. Other eye tracking systems may use non-contact optical measurements of eye motion.

In another example, the subject sensor data may comprise subject pupil width. The subject pupil width may for example be measured by using a camera examining or monitoring the subject's face. This for example could be done by an external camera or it may even be performed by a camera being used by a teleconferencing system by the subject.

In another example, the subject sensor data comprises subject skin conductivity. Measuring the subject skin conductivity may be a good measure of the person's level of stress or physical exertion. The subject sensor system may be configured for monitoring the subject skin conductivity.

In another example, the subject sensor data comprises subject physical activity. For example, the subject sensor system may be a carried or wrist worn device with an accelerometer used for measuring the physical activity of the subject. The subject's physical activity could for example be a current level of physical activity or the average physical activity within a predetermined amount of time.

In another example, the subject sensor data further comprises subject average physical activity.

In another example, the subject sensor data further comprises subject heart rate. This may for example be a good measure of the subject's stress or current activity also.

In another example, the subject sensor data further comprises subject blood pressure.

In another example, the subject sensor data further comprises subject blood oxygen level.

In another example, the subject sensor data further comprises subject surrounding noise environment classification. For example, a handheld device with a microphone could be used to measure the surrounding noise environment of the subject and then this could for example be classified using a neural network or analytical algorithm. This may for example be a good measure of providing insight as to how much of a distraction is surrounding the subject in the environment.

In another example, the subject sensor data further comprises subject speech volume level. The subject sensor for example could be a microphone incorporated into a smartphone or wearable device which measures the speech volume of the subject. This may also be a good indicator of how receptive the subject could be to the medical message.

In another example, the subject sensor data further comprises detection of subject swearing. For example, an audio-to-speech detector may be used to detect certain keywords such as those which would indicate stress or distress. This may be useful in modifying the medical message to fit the state of the subject.

In another example, the subject sensor data further comprises the detection of subject singing. This may for example include such things as humming or a greater than average modulation in the pitch of the subject's voice. This may for example be used to indicate when the subject is in a good mood or has a receptive mood for receiving information.

In another example, the subject sensor data further comprises a duration of subject reading a text. This may for example be useful in determining the concentration or time that a subject can focus on reading a medical message. If the duration for example is lower than average then this may be an indication to reduce the size of the medical message and reduce it only to important parts. The duration of the subject reading a text may for example be used by the same system which is used to measure subject eye motion data.

In another example, the medical system further comprises a configuration database. A database as used herein may encompass such things as a file which contains data, a spreadsheet table, or even a relational database. The configuration database stores the predetermined criterion referenced by a subject-specific identifier. The subject-specific identifier may mean that the predetermined criterion is configured differently for different subjects. Execution of the machine-executable instructions further causes the computational system to receive the subject-specific identifier. Execution of the machine-executable instructions further causes the computational system to retrieve the predetermined criterion in response to querying the configuration database with the subject-specific identifier. This example may be beneficial because it may provide for a means of personalizing the control signal for a particular individual.

In another example, execution of the machine-executable instructions further causes the computational system to receive additional subject sensor data after transmission of the message to the subject. Execution of the machine-executable instructions further causes the computational system to determine a communication score by comparing the additional subject sensor data to a baseline criterion. For example, the measure of how long a subject is reading a the text or some other measure of concentration may be compared to this baseline criterion and this may be useful in determining how effective the communication was with the subject. Execution of the machine-executable instructions further causes the computational system to update the predetermined criterion using the additional subject sensor data if the communication score is outside a predetermined value. Updating the predetermined criterion may be an effective means of dealing with the case when the medical message was not determined to be ineffective as determined by the communication score.

In another example, execution of the machine-executable instructions further causes the computational system to provide pre-generated text as at least a portion of the medical message and to modify the pre-generated text using the message text. The pre-generated text may be provided in several different ways. In one example templated text for a particular type of medical examination or imaging protocol may be provided as a base to be edited. In other examples an artificial intelligence such as a large language model (LLM) may be queried and used to provide the pre-generated text. Modifying the pre-generated text may be beneficial because it may then be fit to the particular situation or case of the subject.

A large language model (LLM) as used herein encompasses a neural network architecture, typically built up of transformers (encoders and decoders) with self-attention layers and residual connections, that is a language model that has been trained using unlabeled text using self-supervised or semi-supervised learning. Typically, LLMs are trained using billions of words. LLMs may for example be trained in an autoregressive form where given a segment of text the model predicts the next word (token) or words (tokens). Another mode of training is where words or tokens within a sentence are missing and the LLM predicts the missing words or tokens. Both types of LLMs may be configured to be used in the so-called prompting paradigm where a text query or statement is input into the LLM and the LLM outputs a completion or statement. The LLMs described herein are configured to operate in the prompting paradigm. Example LLMs are GPT-3, GPT-4, BERT, LLaMA, and others. The LLM may be trained for specific tasks using reinforcement learning or by reinforcement learning from human feedback (RLHF). The output of a preexisting LLM may be adjusted using fine-tuning. In fine-tuning a new set of weights connecting the final layer of the language model may be trained with specific data. Typically, this is done by freezing the other weights (other than the final output layer) in the neural network so that only the final output and format is affected.

In this particular example, the LLM may be trained by collection exemplary text data and pairing it with feedback generated by a human and training it with RLHF.

In another example, the message metadata comprises cursor motion data. This may for example measure how the cursor of the user interface is moved. The cursor going back in different locations may indicate that the subject is spending more time on drafting the text.

In another example, the message metadata further comprises mouse tracking data. Likewise, monitoring how the mouse is moving may indicate how much care a subject is spending on editing the text.

In another example, the message metadata further comprises a duration spent reading.

In another example, the message metadata further comprises comparison with templated text data. This may for example be a useful means of checking historical texts against the current one.

In another example, the message metadata further comprises operator eye motion data descriptive of reading by the operator.

In another example, the message metadata further comprises the operator pupil width.

In another example, the message metadata further comprises the operator skin conductivity.

In another example, the message metadata further comprises operator physical activity.

In another example, the message metadata further comprises operator average physical activity.

In another example, the message metadata further comprises the operator heart rate.

In another example, the message metadata further comprises operator blood pressure.

In another example, the message metadata further comprises operator blood oxygen level.

In another example, the message metadata further comprises operator surrounding noise environment classification.

In another example, the message metadata further comprises operator speech volume level.

In another example, the message metadata further comprises the detection of operator swearing.

In another example, the message metadata further comprises the detection of operator singing.

In another example, the comparison of the subject sensor data and the message metadata to the predetermined criterion is made using mathematical inequalities to check range specified in the predetermined criterion.

In another example, the comparison of the subject sensor data and the message metadata is made using an expert system. In this example, the contents of the expert system provide the predetermined criterion. The expert system could for example be configured by collecting data from many sessions when the message text is being comprised and then using this data to construct the expert system.

In another example, the comparison of the subject sensor data and the message metadata is made using a neural network. For example, the neural network may be a classifier neural network.

A classifier neural network may be a neural network such as a neural network with multiple (at least 3) fully connected layers or a convolutional neural network.

The neural network could be trained by collecting groups of subject sensor data and message metadata and then manually generated control signals. The combination of the subject sensor data and message metadata with the control signal may form training data which may be used for example for a deep learning process.

In another example, the control signal is configured for displaying a warning message.

In another example, the control signal is configured for modifying an appearance of a portion of the medical message to be edited further. For example, the medical message could have a region which is highlighted and should indicate to the author to edit the text further.

In another example, the control signal is configured to highlight changes in the medical message. The highlighted portions for example may cause a difference in the coloration behind the text, or a change in the size or appearance of the text so that it is apparent to the operator.

In another example, the control signal is configured to display the percentage of the medical message edited. This may for example be useful for the operator to know how much the text has been changed.

In another example, the control signal is configured to disable sending of the medical message until the predetermined criterion is satisfied. For example, if the operator did not modify any existing or templated text, then the transmission of the medical message could be prevented.

In another example, the control signal is configured to append the message metadata to the medical message. This for example may be useful for the recipient of the medical message to understand the state or context of the medical message.

In another example, the control signal is configured to generate message notes appended to the medical message. This may for example be used to label the medical message and indicate portions which the recipient should have more or less confidence in.

In another example, the message comprises at least one medical image. The message text comprises at least one annotation of the medical image. This example may be beneficial because the annotation of medical images by radiologists is an important task and is also subject to a high degree of variability depending upon the recipient of the medical message as well as the state of the person doing the annotation.

In another example, the medical system further comprises a medical imaging system configured for acquiring the at least one medical image according to a medical imaging protocol. The predetermined criterion is at least partially determined by the medical imaging protocol. This may be useful for example the medical protocol may be useful for specifying certain criteria or standards in the production of the medical message.

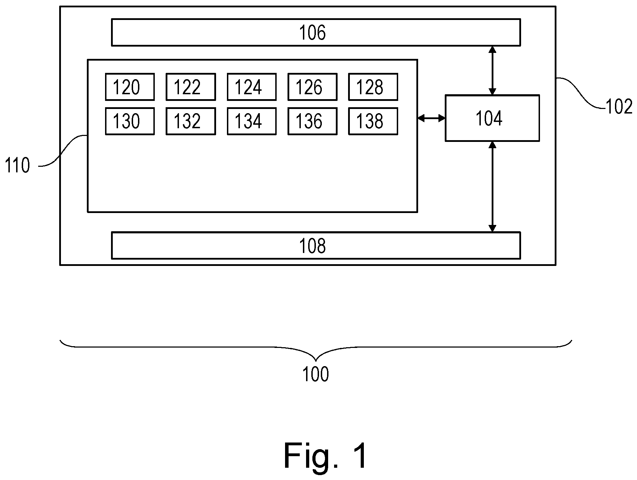

Fig. 1 illustrates an example of a medical system 100. The medical system 100 is shown as comprising a computer 102. The computer 102 may represent on or more computers in one or more locations. The computer 102 comprises a computational system 104. Likewise, the computational system 104 may represent one or more computing or computational cores located at one or more locations. The medical system 100 is further shown as comprising a hardware interface and/or network interface 106 that may for example be used to control other components of the medical system 100 if they are present or it can communicate with other computational or computer systems.

The hardware interface 106 is shown as being in communication with the computational system 104. The computational system 104 is in further communication with a user interface 108 and a memory 110. The user interface 108 may for example comprise one or more human interface devices and be configured for receiving message text from an operator. The memory 110 may represent different types of memory that are accessible to the computational system 104. In some examples the memory 110 may comprise a non-transitory storage medium.

The memory 110 is shown as containing machine-executable instructions 120. The machine-executable instructions 120 may comprise instructions which enable the computational system 104 to perform tasks such as manipulation of text, data, and control other components of the medical system 100. The memory 110 is further shown as containing an optional configuration database 122. The configuration database 122 may for example be configured to receive a subject-specific identifier 124 and in response provide or output a predetermined criterion 126. The memory 110 is shown as containing an optional subject-specific identifier 124 and the predetermined criterion 126. In some examples the predetermined criterion 126 may already be stored in the memory 110. The subject-specific identifier 124 is used to identify a particular subject.

The memory 110 is further shown as containing subject sensor data 128 that was measured from a subject. It for example could be received from a subject sensor system in communication with the hardware interface 106 or it may be retrieved from a remote location where the subject is located. The memory 110 is further shown as containing a message text 130 that has been entered or is being worked on by the operator of the medical system 100 using the user interface 108. The memory 110 is further shown as containing an optional text template 132. The message text 130 could for example contain part of or all of the text template 132. In some examples the medical system 100 may be for modifying or personalizing the text template 132. The memory 110 is further shown as containing message metadata 134 that was recorded while the message text 130 was entered by the operator. This may contain such things as the usage of the user interface 108 as well as additional sensor data from sensor measurements made directly on the operator in addition. The memory 110 is further shown as containing an optional control module 136. The optional control module 136 takes as input the predetermined criterion 126 and this is compared against the subject sensor data 128 and the message metadata 134 and in response produces a control signal 138. The control signal 138 is used to modify the behavior of the user interface 108 for further reception of the message text 130. In other instances the control signal 138 is used to modify or change the message text 130 when constructing a medical image. The memory 110 is further shown as containing the medical message 140 that was generated at least partially using the message text 130.

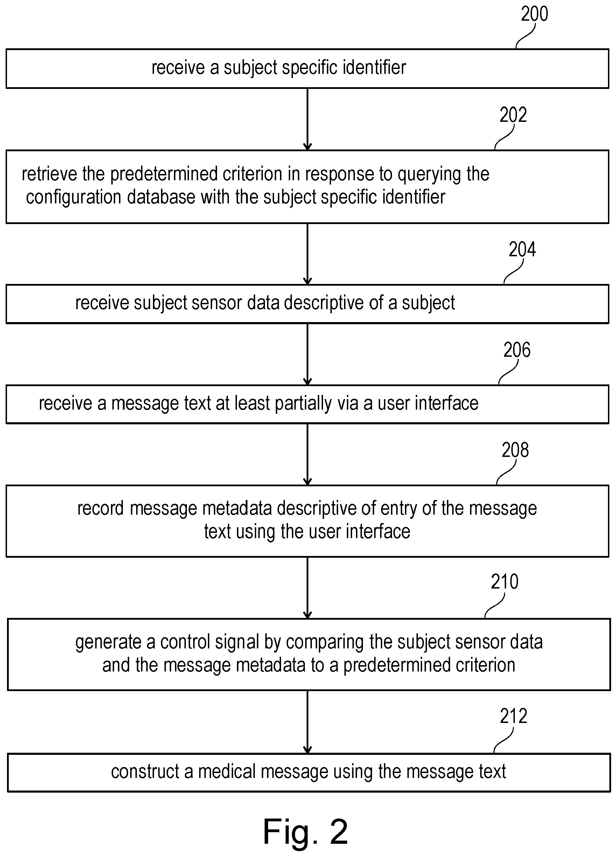

Fig. 2 shows a flowchart which illustrates a method of operating the medical system 100 of Fig. 1. In optional step 200 the subject-specific identifier 124 is received. In optional step 202 the predetermined criterion 126 is received in response to querying the configuration database 122 with the subject-specific identifier 124. In step 204, the subject sensor data 128 is received. The subject sensor data 124 is descriptive of a subject. In step 206 the message text 130 is received at least partially via the user interface 108. In step 208 the message metadata 134 is recorded during entry of the message text 130 using the user interface 108. The message metadata 134 is descriptive of the entry of the message text 130 using the user interface 108. In step 210, the control signal 138 is generated by comparing the subject sensor data 128 and the message metadata 134 to a predetermined criterion 126. Then, in step 212, the medical message 140 is constructed using the message text 130. For example, the medical message may be an annotated medical image and the message text 130 may be inserted to or used to label portions of the medical image.

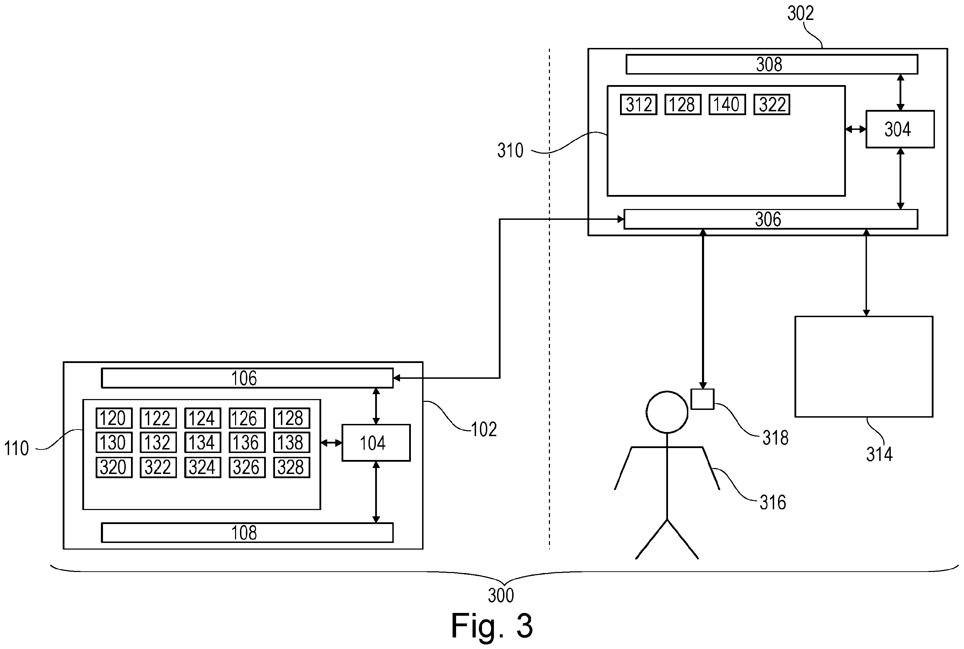

Fig. 3 illustrates a further example of a medical system 300. The medical system 300 is similar to that depicted in Fig. 1 except that it additionally comprises a number of components used by a message subject 316. The message subject 316 is the subject that is the intended recipient of the medical message 140. There is a subject computer 302 that comprises a subject computational system 304. The subject computational system 304 is in communication with a subject network interface 306, a subject user interface 308, and a subject memory 310. The subject network interface 306 is in communication with the network interface 106 of the computer 102. In some examples the features of the computer 102 and the subject computer 302 are combined.

The network or subject interface 306 is in communication with a subject display unit 314 and a subject sensor system 318. The subject display unit 314 may be used for displaying the medical message 140 to the subject 316. The subject sensor system 318 may be a sensor which may be used to measure a variety of physical or physiological measurements on the subject 316. The subject sensor data 128 may be recorded or measured using the subject sensor system 318.

The subject memory 310 is shown as comprising or storing the subject machine-executable instructions 312 which enable the subject computational system 304 to process data and communicate with the computational system 104. The subject memory 310 is further shown as containing the subject sensor data 128 that was measured with the subject sensor system 318 and then transmitted to the computational system 104. The subject memory 310 is further shown as containing the medical message 140 that was received from the computational system 104. The subject memory 310 is further shown as containing additional subject sensor data 322 that was recorded when the medical message 140 was displayed to the subject 316. The additional subject sensor data 322 was also transmitted back to the computational system 104 and stored in the memory 110. The computational system 104 then also gave a communication score 324 to the additional subject sensor data 322 and compared it to a predetermined value 326. If the communication score 324 is outside of the predetermined value 326, then the predetermined criterion 126 may be updated for the subject 316. This may for example provide for a new baseline.

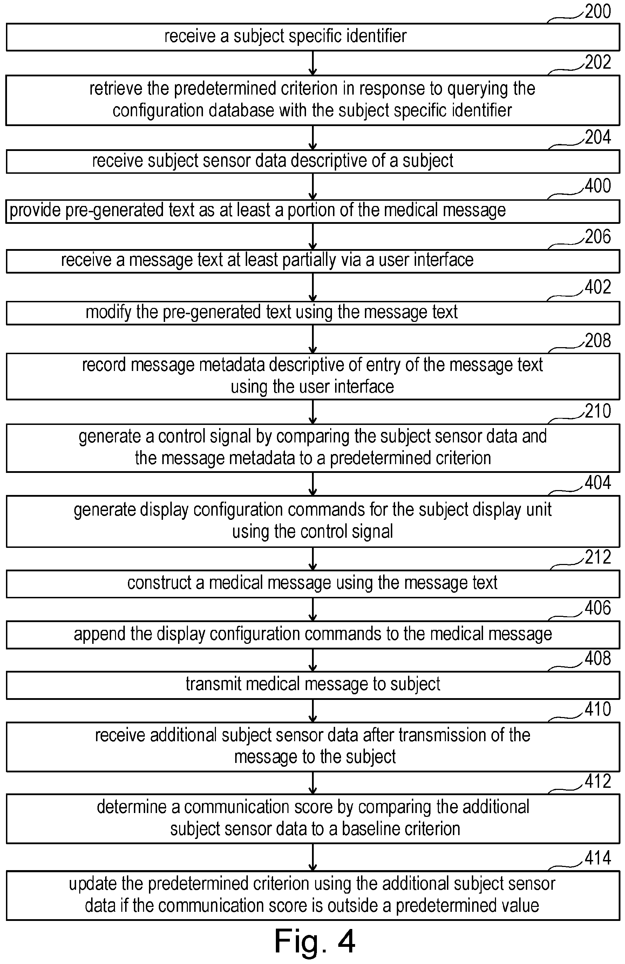

Fig. 4 shows a flowchart which illustrates a method of operating the medical system 300 of Fig. 3. Steps 200, 202, and 204 are performed as was illustrated in Fig. 2. In step 400 a pre-generated text 328 is provided. This pre-generated text 328 may be used as a portion of the medical message 140. Step 206 is performed as was illustrated in Fig. 2. In step 402 the pre-generated text 328 is modified using the message text 130. Steps 208 and 210 are performed as was illustrated in Fig. 2. In step 404 display configuration commands 320 were generated for the subject display 314 using the control signal 138. Step 212 is performed as was illustrated in Fig. 2. In step 406 the display configuration commands 320 are appended to the medical message 140. In step 408 the medical message 140 is transmitted to the subject computational system 304. While the message is displayed on the subject display unit 314, the additional subject sensor data 322 is recorded. This is then transmitted from the subject computational system 304 to the computational system 104. In step 410 the additional subject sensor data 322 is received. In step 412 a communication score 324 is determined by comparing the additional subject sensor data to a baseline criterion. In step 414 the predetermined criterion 126 is updated using the additional subject sensor data 322 if the communication score 324 is outside of the predetermined value 326.

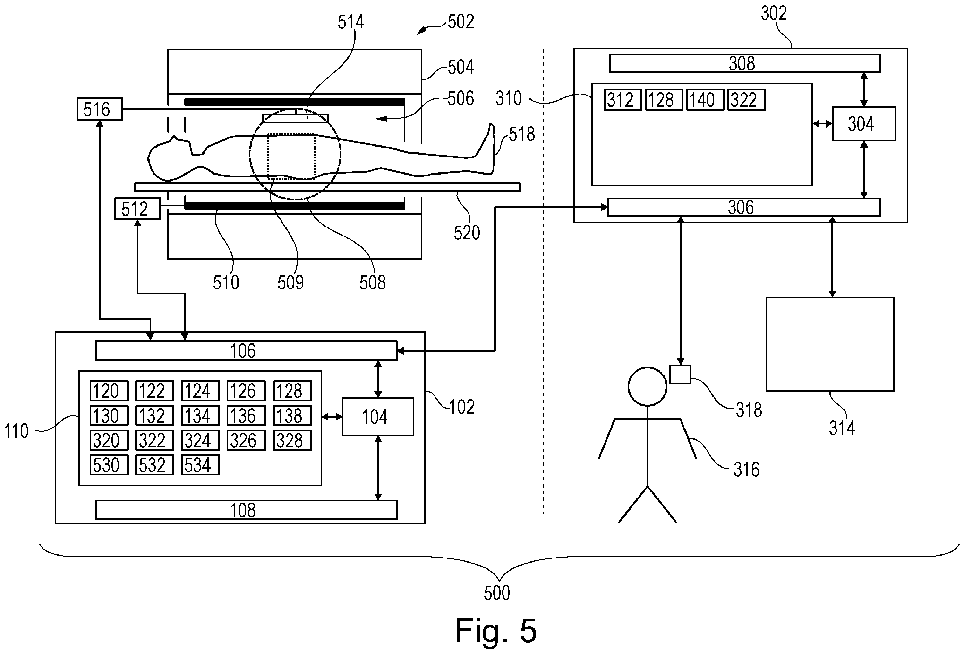

Fig. 5 illustrates a further example of the medical system 500. The medical system 500 illustrated in Fig. 5 is very similar to the medical system 300 illustrated in Fig. 3 except that it additionally comprises a magnetic resonance imaging system 502.

The magnetic resonance imaging system 502 comprises a magnet 504. The magnet 504 is a superconducting cylindrical type magnet with a bore 506 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 506 of the cylindrical magnet 504 there is an imaging zone 508 where the magnetic field is strong and uniform enough to perform magnetic resonance imaging. A field of view 509 is shown within the imaging zone 508. The k-space data are acquired for the field of view 509. The region of interest could be identical with the field of view 509 or it could be a sub volume of the field of view 509. A subject 518 is shown as being supported by a subject support 520 such that at least a portion of the subject 518 is within the imaging zone 508 and the field of view 509.

Within the bore 506 of the magnet there is also a set of magnetic field gradient coils 510 which is used for the acquisition of measured k-space data to spatially encode magnetic spins within the imaging zone 508 of the magnet 504. The magnetic field gradient coils 510 are connected to a magnetic field gradient coil power supply 512. The magnetic field gradient coils 510 are intended to be representative. Typically, magnetic field gradient coils 510 contain three separate sets of coils for spatial encoding in three orthogonal spatial directions. A magnetic field gradient power supply supplies current to the magnetic field gradient coils. The current supplied to the magnetic field gradient coils 510 is controlled as a function of time and may be ramped or pulsed.

Adjacent to the imaging zone 508 is a radio frequency coil 514 for manipulating the orientations of magnetic spins within the imaging zone 508 and for receiving radio transmissions from spins also within the imaging zone 508. The radio frequency antenna may contain multiple coil elements. The radio frequency antenna may also be referred to as a channel or antenna. The radio frequency coil 514 is connected to a radio frequency transceiver 516. The radio frequency coil 514 and radio frequency transceiver 516 may be replaced by separate transmit and receive coils and a separate transmitter and receiver. It is understood that the radio frequency coil 514 and the radio frequency transceiver 516 are representative. The radio frequency coil 514 is intended to also represent a dedicated transmit antenna and a dedicated receive antenna. Likewise, the transceiver 516 may also represent a separate transmitter and receiver. The radio frequency coil 514 may also have multiple receive/transmit elements and the radio frequency transceiver 516 may have multiple receive/transmit channels. The transceiver 516 and the gradient controller 512 are shown as being connected to the hardware interface 106 of the computer system 102.

The memory 110 is further shown as containing pulse sequence commands 530. The pulse sequence commands are commands or data which may be converted into commands for controlling the magnetic resonance imaging system 502 to acquire k-space data 532. The memory 110 is further shown as containing k-space data 532 that was acquired by controlling the magnetic resonance imaging system 502 with the pulse sequence commands 530. The memory 110 is further shown as containing a magnetic resonance image 534 that was reconstructed from the k-space data 532. The medical message 140 in this case also comprises the magnetic resonance image 534. For example, the medical message 140 could be a DICOM image container or message. The message text 130 could be annotations of the magnetic resonance image 534 or could be text describing the results or the anatomy present in the magnetic resonance image 534. For the example illustrated in Fig. 5 the subject 518 could be an examination subject and then the subject 316 could be the recipient of the medical message 140. For example, the subject 316 could be a doctor or other healthcare professional who had requested the magnetic resonance image 534.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface / network interface
- 108: user interface
- 110: memory
- 120: machine executable instructions
- 122: configuration database
- 124: subject specific identifier
- 126: predetermined criterion
- 128: subject sensor data
- 130: message text
- 132: text template
- 134: message metadata
- 136: control module
- 138: control signal
- 140: medical message
- 200: receive the subject specific identifier
- 202: retrieve the predetermined criterion in response to querying the configuration database with the subject specific identifier
- 204: receive subject sensor data descriptive of a subject
- 206: receive a message text at least partially via a user interface
- 208: record message metadata descriptive of entry of the message text using the user interface
- 210: generate a control signal by comparing the subject sensor data and the message metadata to a predetermined criterion
- 212: construct a medical message using the message text
- 300: medical system
- 302: subject computer
- 304: subject computational system
- 306: subject hardware interface / network interface
- 308: subject user interface
- 310: subject memory
- 312: subject machine executable instructions
- 314: subject display unit
- 316: message subject
- 318: subject sensor system
- 320: display configuration commands
- 322: additional subject sensor data
- 324: communication score
- 326: predetermined value
- 328: pregenenoterated text
- 400: provide pre-generated text as at least a portion of the medical message
- 402: modifying the pre-generated text using the message text
- 404: generate display configuration commands for the subject display unit using the control signal
- 406: append the display configuration commands to the medical message, wherein the subject display unit is configured to modify display of the medical message using the display configuration commands to transmit the medical message to the subject
- 408: transmit medical message to subject
- 410: receive additional subject sensor data after transmission of the message to the subject
- 412: determine a communication score by comparing the additional subject sensor data to a baseline criterion
- 414: update the predetermined criterion using the additional subject sensor data if the communication score is outside a predetermined value
- 500: medical system
- 502: magnetic resonance imaging system
- 504: magnet
- 506: bore of magnet
- 508: imaging zone
- 509: field of view
- 510: magnetic field gradient coils
- 512: magnetic field gradient coil power supply
- 514: radio frequency coil
- 516: transceiver
- 518: examination subject
- 520: subject support
- 530: pulse sequence commands
- 532: k-space data
- 534: magnetic resonance image

## Claims

1. A medical system (100, 300, 500) comprising:
- a memory (110) storing machine executable instructions (120);
- a computational system (104), wherein execution of the machine executable instructions causes the computational system to:
- receive (204) subject sensor data (128) descriptive of a subject (316);
- receive (206) a message text (130) at least partially via a user interface (108);
- record (208) message metadata (134) descriptive of entry of the message text using the user interface;
- generate (210) a control signal (138) by comparing the subject sensor data and the message metadata to a predetermined criterion (126), and wherein the control signal is configured to modify the user interface for further entry or modification of the message text; and
- construct (212) a medical message (140) using the message text.

2. The medical system of claim 1, wherein execution of the machine executable instructions further causes the computational system to transmit (408) the medical message to the subject.

3. The medical system of claim 1 or 2, wherein the medical system further comprises a subject display unit (314), wherein transmitting the medical message to the subject comprises displaying the medical message on the subject display unit, wherein execution of the machine executable instructions further causes the computational system to:
- generate (404) display configuration commands for the subject display unit using the control signal; and
- append (406) the display configuration commands to the medical message, wherein the subject display unit is configured to modify display of the medical message using the display configuration commands.

4. The medical system of any one of claim 1, 2, or 3, wherein the medical system further comprises a subject sensor system (318) configured for measuring the subject sensor data and for transmitting the subject sensor data to the computational system.

5. The medical system of any one of the preceding claims, wherein the subject sensor data comprises any one of the following: subject eye motion data descriptive of reading by the subject, subject pupil width, subject skin conductivity, subject physical activity, subject average physical activity, subject heart rate, subject blood pressure, subject blood oxygen level, subject surrounding noise environment classification, subject speech volume level, detection of subject swearing, detection of subject singing, duration of subject reading a text, and combinations thereof.

6. The medical system of any one of the preceding claims, wherein the medical system further comprises a configuration database (122), wherein the configuration database stores the predetermined criterion referenced by a subject specific identifier (124), wherein execution of the machine executable instructions further causes the computational system to:
- receive (200) the subject specific identifier; and
- retrieve (202) the predetermined criterion in response to querying the configuration database with the subject specific identifier.

7. The medical system of claim 6, wherein execution of the machine executable instructions further causes the computational system to:
- receive (410) additional subject sensor data (322) after transmission of the message to the subject;
- determine (412) a communication score (324) by comparing the additional subject sensor data to a baseline criterion; and
- update (414) the predetermined criterion using the additional subject sensor data if the communication score is outside a predetermined value.

8. The medical system of any one of the preceding claims, wherein execution of the machine executable text further causes the computational system to:
- provide (400) pre-generated text as at least a portion of the medical message; and
- modifying (402) the pre-generated text using the message text.

9. The medical system of any one of the preceding claims, wherein the message metadata comprises any one of the following: cursor motion data, mouse tracking data, duration spend reading, comparison with templated text data, operator eye motion data descriptive of reading by the subject, operator pupil width, operator skin conductivity, operator physical activity, operator average physical activity, operator heart rate, operator blood pressure, operator blood oxygen level, operator surrounding noise environment classification, operator speech volume level, detection of operator swearing, detection of operator singing, and combinations thereof.

10. The medical system of any one of the preceding claims, wherein the comparison of the subject sensor data and the message metadata to the predetermined criterion is made using any one of the following: mathematical inequalities to check ranges specified in the predetermined criterion, using an expert system, using a neural network, and combinations thereof.

11. The medical system of any one of the preceding claims, wherein the control signal is configured for any one of the following: display a warning message, modify an appearance of a portion of the medical message to be edited further, highlight changes in the medical message, display a percentage of the medical message edited, disable sending of the medical message until the predetermined criterion is satisfied, append the message metadata to the medical message, generate message notes appended to the medical message, and combinations thereof.

12. The medical system of any one of the preceding claims, wherein the message comprises at least one medical image (534), and wherein the message text comprises at least one annotation of the medical image.

13. The medical system of claim 12, wherein the medical system further comprises a medical imaging system (502) configured for acquiring the at least one medical image according to a medical imaging protocol, and wherein the predetermined criterion is at least partially determined by the medical imaging protocol.

14. A method, wherein the method comprises:
- receiving (204) subject sensor data (128) descriptive of a subject (316);
- receiving (206) a message text (130) at least partially via a user interface (108);
- recording (208) message metadata (134) descriptive of entry of the message text using the user interface;
- generating (210) a control signal (138) by comparing the subject sensor data and the message metadata to a predetermined criterion (126), and wherein the control signal is configured to modify the user interface for further entry or modification of the message text; and
- constructing (212) a medical message using the message text.

15. A computer program comprising machine executable instructions (120), wherein execution of the machine executable instructions causes a computational system (104) to:
- receive (204) subject sensor data (128) descriptive of a subject (316);
- receive (206) a message text (130) at least partially via a user interface;
- record (208) message metadata (134) descriptive of entry of the message text using the user interface;
- generate (210) a control signal (138) by comparing the subject sensor data and the message metadata to a predetermined criterion (126), and wherein the control signal is configured to modify the user interface for further entry or modification of the message text; and
- construct (212) a medical message (140) using the message text.
